# EUROPEAN PATENT APPLICATION

(11) **EP 3 760 701 A1**
(43) Date of publication of application: **06.01.2021**
(21) Application number: 19761426.6
(22) Date of filing: 26.02.2019
(51) Int. Cl.: C12M 1/00, C12M 3/00

(54) **CELL HOLDING AND TRANSPORTING DEVICE**

(30) Priority: 28.02.2018 JP 2018035458
(71) Applicant: Stem Cell & Device Laboratory, Inc., Kyoto-shi, Kyoto 600-8491 (JP)
(72) Inventor: HORI Kosuke, Kyoto-shi Kyoto 600-8491 (JP); KAWAHARA Noriyuki, Kyoto-shi Kyoto 600-8491 (JP)
(74) Representative: Kalhammer, Georg
(86) International application number: PCT/JP2019/007361
(87) International publication number: WO 2019/167960

(57) **Abstract**

[Problems]

A devise for retaining and transporting cells thatis capable of holding cultured cells and moving the cells while holding them is provided.

[Solutions]

The device for retaining and transporting cells 100 has a base 101, a through hole 103, a cell scaffold 105, and an manipulating part 107. The cell scaffold 105 holds cells such as cardiac myocytes and nerve cells. When holding the cells in the cell scaffold 105, drop the suspension from the through hole 103 to the cell scaffold 105 exposing from the through hole 103 using a pipette etc. and culture. The manipulating part 107 is formed so as to project from the upper surface P101a of the base 101. Multiple manipulating part 107 is formed radially on the upper surface P101a of the base 101, centered on the through hole 103. By forming the manipulating part 107, the manipulating part 107 can be held by a holder such as tweezers, which enables easy operation of the device for retaining and transporting cells 100.

## Description

### [Technical Field]

The present invention relates to a device for retaining and transporting cells, and particularly, to a device facilitating the transportation of the retained cells.

### [Background Art]

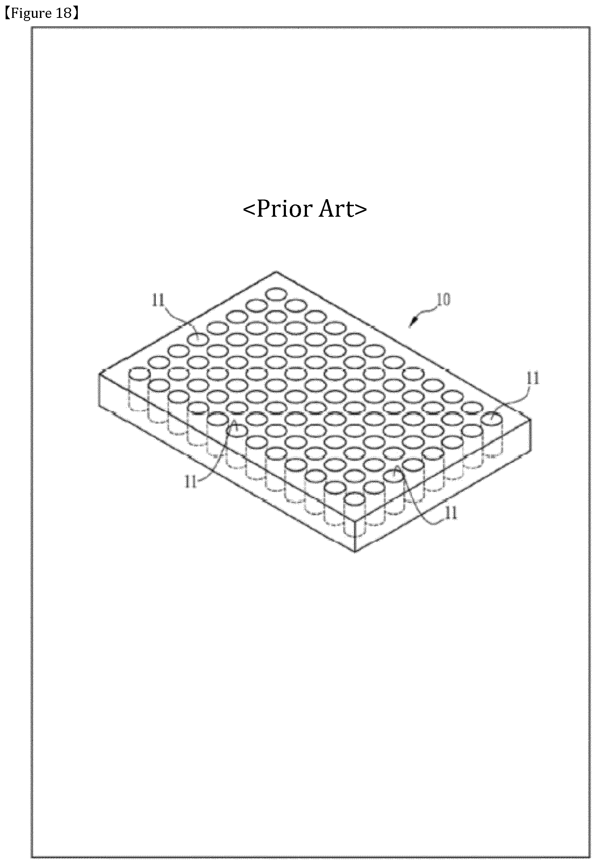

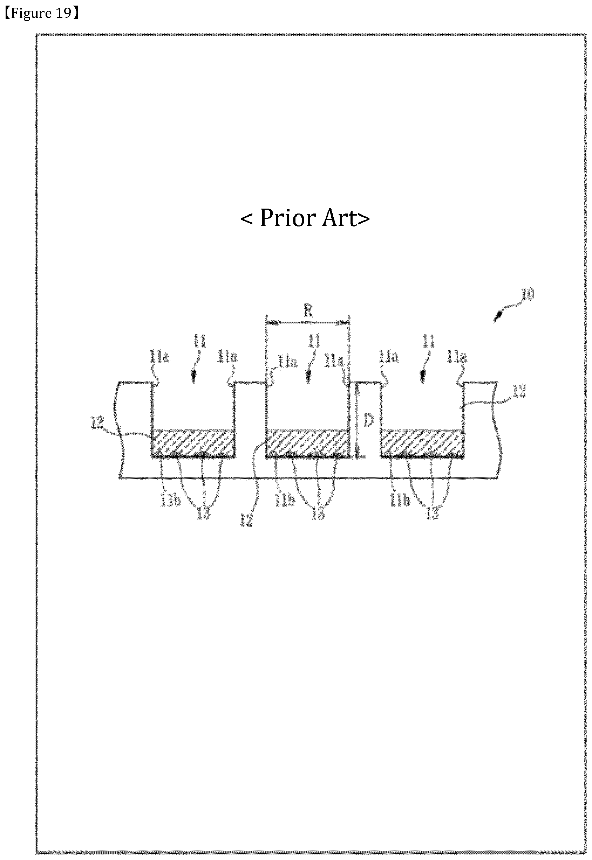

To illustrate the conventional technique relating to the device for retaining and transporting the cells, plastic container 10 is used. As shown in Fig. 18, the plastic container 10 is a vessel to culture living cells such as cells and tissues of living organisms, and to capture images of (or observe) the cultured living cells with a phase contrast microscope. The plastic container 10 is also referred to as a multi-well plate having a plurality of well, 11, with round openings. The well 11 is a recess to accommodate the living cells to be imaged after the culture as well as the culture medium for cultivating the living cells. Fig. 19 shows, as an example, a 96 well plate having ninety-six wells of the well 11 in eight rows and twelve columns. Number of the wells in the plastic container 10 is arbitrary as long as it is equal to or more than two. In addition to the 96 well plate, multi-well plates having six, twenty-four, or three hundred eighty-four wells of the well 11 are often used.

Suitable materials for the plastic container 10 are, for example, polyethylene terephthalate (PET), polystyrene (PS), polycarbonate (PC), TAC (triacetyl cellulose), polyimide (PI), nylon (Ny), low-density polyethylene (LDPE), medium-density polyethylene (MDPE), vinyl chloride, vinylidene chloride, polyphenylene sulfide, polyether sulfone, polyethylene naphthalate, polypropylene, acrylic tmaterials such as urethane acrylate, cellulose, glass and the like. In addition, resins including biodegradable polymers such as polylactic acid, polyglycolic acid, polycaprolactane, or copolymers thereof can be also used. Among these, polyethylene terephthalate, polystyrene, and polycarbonate are preferably used, and polystyrene are particularly preferable because of their low cytotoxicity. The surface of the plastic container 10 can be subjected to any surface treatment (for example, irradiation with plasma, corona, microwave, electron beam, ultraviolet ray, or the like).

As shown in Fig. 19, the well 11 is a non-through hole and is opened on the surface of the plastic container 10. Through the opening, to the well 11 are added living cells 13 to be cultured and a culture solution 12 (for example, serum) to culture the living cells 13. In the case of using the plastic container 10, wherein the well 11 is opened, the culture solution 12 in the well 11 is constantly in contact with air in the vicinity of the opening of the well 11 both during the cell culture and in phase contrast microscopic analysis of the living cells 13. Therefore, the cultured living cells 13 are imaged (observed) with the phase-contrast microscope while being alive.

In the plastic container 10, the well 11 is formed with an opening diameter R of 0.5 mm to 2 cm, and a depth D of 2 mm to 2 cm. The opening diameter R of the well 11 is preferably 0.5 mm to 2 cm, and more preferably 1 mm or more, is to obtain the data converging the amount of the living cell 13 cultured under the same environment without variation. When the opening diameter R of the well 11 is less than 2 cm, the meniscus of the liquid surface 12a of the culture solution 12 becomes conspicuous, thereby making the present invention particularly useful. The opening diameter R of the well 11 of 1 cm or less is particularly preferable. For example, in the case of a 96 well plate having ninety-six wells of well 11 and a 384 well plate having three hundred eighty-four wells of the well 11, the opening diameters of the well 11 are 6 mm and 3 mm, respectively.

The depth D of the well 11 refers to the height from the bottom 11b of the well 11 to the opening (the surface of the plastic container 10). The depth D of the well 11 preferably equal to or more than 2 mm to retain a sufficient amount both of medium and of the culture solution 12 to culture the living cell 13. The depth D of the well 11 is preferably equal to or less than 2 cm to suppress the decrease in the light intensity in the periphery of visual field due to vignetting in transmission microscopy. The depth D of the well 11 of3 mm equal to or less than 1 cm is particularly preferable for culturing and imaging the living cells 13. Also, the amount of the culture solution 12 to be added to the well 11 is preferably equal to or less than a half of the depth D of the well 11 (see Patent Document 1).

### [Precedent Technical Literature]

### [Patent Document]

[Patent Document 1] JP-A 2016-67322

### [Summary of Invention]

### [Issues Invention Is to Address]

The said plastic container 10 has the following issues to be solved. When performing experiments using the said living cell 13, it is necessary to culture the living cell 13 directly in the well 11 of the plastic container 10 in advance. As a result, the experiment and the cell culture cannot be separated. This increases the burden on the experimenter because, even if the main purpose is to conduct the experiment on the living cell 13, the living cell 13 must be cultured in advance, which means that the know-how for culturing the living cell 13 is required in addition to the know-how of the experiment on the living cell 13. In particular, the burden on the experimenter is greater when performing an experiment using a plurality of cell lines of the living cell 13.

Also, it is inefficient to conduct live cell culture and experiments in a sequential manner in the laboratory.

In addition, the living cell 13 cultured in the well 11 of the plastic container 10 cannot be replaced, even if a defect occurs in a part thereof, because it is cultured directly on the well 11.

The living cell 13 is cultured on the bottom of the well 11 in the plastic container 10. Because the area of the bottom of the well 11 is relatively large compared to the cells to be cultured, it is unclear where and to what extent the cells are cultured. Therefore, the cells are not uniformly cultured on the bottom of the well 11 even if the prerequisites for cell culture, amount of cells, amount of the medium, temperature, and other conditions are identical. Result may vary depending on the position of the cells in the well 11 subjected to the experiment.

Furthermore, for ensuring the cell culture on the bottom of the well 11 so as to secure the amount of cells needed for testing and the like, the amount of cells is greater than the amount originally needed, which results in the cost increase.

Based on the above background, this invention aims at providing a device for retaining and transporting cultured cells, a method for measurement of the characteristics of the cells using the said device, and a kit for cell characterization including the said device.

That is, the present invention provides a solution to the said issues by the following inventions described in the items (1) to (15):
(1) A device arranged in a cell culture container for retaining and transporting cells, which comprises a base with an upper and a lower surface;
   a through hole formed so as to penetrate the said base;
   a cell scaffold to hold the cells located along the lower surface of the said base; and
   a manipulating part that can be held by a specified holding tool.
(2) The device according to (1), which further comprises a medium storage space forming part that forms a medium storage space above the said through hole.
(3) The device according to (1) or (2),
   wherein the said manipulating part is protruding from the upper surface of the said base.
(4) The device according to (1) or (2), wherein the said manipulating part is formed concaving from the upper surface of the said base.
(5) The device according to any one of (1) to (4), which further comprises an air vent penetrating the said base.
(6) The device according to any one of (1) to (5), wherein the said through hole has a shape narrowing from the surface of the protrusion towards the opposite surface.
(7) The device according to any one of (1) to (6), wherein the said base is formed as a flat plate.
(8) The device according to any one of (1) to (7), wherein the said base is at least made of a material that has larger specific gravity than the medium.
(9) The device according to any one of (1) to (8), wherein the said cell scaffold is a thin fiber sheet.
(10) The device according to (9), wherein the said fiber sheet is prepared by a polymeric material.
(11) The device according to any one of (1) to (10), wherein the said cell scaffold comprises biomaterials.
(12) The device according to (11), wherein the said cell scaffold comprises specified cells.
(13) The device according to (11), wherein the said cell scaffold comprises cardiac myocytes and nerve cells.
(14) A method for measuring cell characterized by using the device according to any one of (1) to (13).
(15) A kit for measuring cell characterized by using the device according to any one of (1) to (13).

### [Advantageous Effects of Invention]

Described below are the means to solve the problems with the present invention, and the effects thereof.

The device of the present invention is a device for retaining and transporting cells arranged in a cell culture vessel, having a flat plate-shaped base with an upper and a lower surface, a through hole formed to penetrate the said base, a cell scaffold located along the lower surface of the base for retaining the cells, and an manipulating part that can be held by a holding tool.

This allows the cultured cells to be retained and transported while being retained.

Separation of the experiment and the cell culture can eliminate the need for the experimenter to do the cell culture, reducing the burden on the experimenter. Because the cell culture can be performed separately to provide the cells to the experimenter, the experimenter can conduct the experiment efficiently. In an event where the cells used in the experiment have a defect, only the defective cells can be easily replaced.

Furthermore, uniform cells can be cultured by focusing on cell culture. Since the cells can be cultured in a limited area, the amount of cells required for the culture can be reduced. Since uniform cells can be cultured, the conditions before the experiment for conducting the comparative experiment and preconditions thereof can be the same.

Furthermore, the user can easily manipulate, for example, move, the device by using a holding tool such as tweezers.

The device of the present invention further comprises a medium storage space forming part that forms a medium storage space above the said through hole.

This allows the medium storage space to be formed above the through hole, so that the medium can be stably stored in the medium storage space. In addition, since additional medium can be stably stored on the base, it is possible to provide fresh medium to the cells retained on the scaffold, and the cells can be stably cultured for a longer period.

The device of the present invention is characterized in that it has manipulating part protruding from the upper surface of the said base.

Therefore, the user can easily manipulate the device using a holding tool such as tweezers.

The device of the present invention is characterized in that the said manipulating part is formed concaving from the upper surface of the said base.

This allows the user to easily manipulate the device by using the holding tool such as tweezers. In addition, the thickness of the device can be reduced.

The device of the present invention comprises an air vent that penetrates the said base.

This allows the air that has entered below the device to be easily expelled to the outside.

The device of the present invention is characterized in that the said through hole has a shape such that it narrows from a surface where the said protrusion is formed towards the opposite surface.

This allows cell suspension to be easily dropped onto the cell scaffold, facilitating the cell culture. In addition, the capacity of the through hole can be increased, allowing a larger amount of the cell suspension to be stored.

The device of the present invention is characterized in that at least the said base is made of a material having a specific gravity heavier than the medium.

This allows the device to be placed easily in the medium. In addition, because of a downward pressure by its own weight, the device can be in close contact with the objects thereunder, such as electrodes or the like.

The device of the present invention is characterized in that the said cell scaffold is a thin fiber sheet.

This makes it easy to produce the device simply by placing the fiber sheet along the lower surface of the base. In addition, the thickness of the device can be made thin and compact.

The device of the present invention is characterized in that the said cell scaffold comprise biomaterials.

This allows the biomaterial to be easily transported, facilitating the experiments using biomaterials.

The device of the present invention is characterized in that the said cell scaffold accommodate cardiac myocytes or nerve cells.

This allows the cardiac myocytes and nerve cells to be easily transported, facilitating the experiments using cardiac myocytes and nerve cells.

### [Brief Description on Figures]

Fig. 1 is a perspective view of a device 100 as an example of the device of the present invention.
Fig. 2 is a plan view of the device 100.
Fig. 3 shows an X1-X1 cross-section of the device 100 shown in Fig.2.
Fig. 4 shows the device 100 in use.
Fig. 5 shows the device 100 in use.
Fig. 6 shows the device 100 in use.
Fig. 7 is a perspective view of a device 200 an example of the device of the present invention.
Fig. 8 is a perspective view of a device 300 as an example of the device of the present invention.
Fig. 9 shows the device 300 in use.
Fig. 10 is a perspective view of a device 400 as an example of the device of the present invention.
Fig. 11 is a perspective view of a device 500 as an example of the device of the present invention.
Fig. 12 is a perspective view of a device 600 as an example of the device of the present invention.
Fig. 13 is a perspective view of a device 700 as an example of the device of the present invention.
Fig. 14 is a perspective view of a device 800 as an example of the device of the present invention.
Fig. 15 is a perspective view of a device 900 as an example of the device of the present invention.
Fig. 16 is a perspective view of a device 1000 as an example of the device of the present invention.
Fig. 17 is a perspective view of a device 1100 as an example of the device of the present invention.
Fig. 18 shows a prior art relating to device for retaining and transporting cells.
Fig. 19 shows a prior art relating to device for retaining and transporting cells.
Fig. 20 is a perspective view of a device 1700 as an example of the device of the present invention.
Fig. 21 shows a cross-section of the device 1700.
Fig. 22 shows a MEA probe P.
Fig. 23 shows the measurement results of the extracellular potential of cardiac myocytes using the device 1700.

### [Modes for Embodying Technology in Disclosure]

Hereinafter, embodiments of this invention are described in detail with reference to the figures.

### [Example 1]

The device of the present invention is described using the device 100 as an example. The device 100 is a device arranged in a well of a 96 well plate for culturing cells in the medium. The cells cultured in the well are used in predefined experiments and tests.

### 1. Configuration

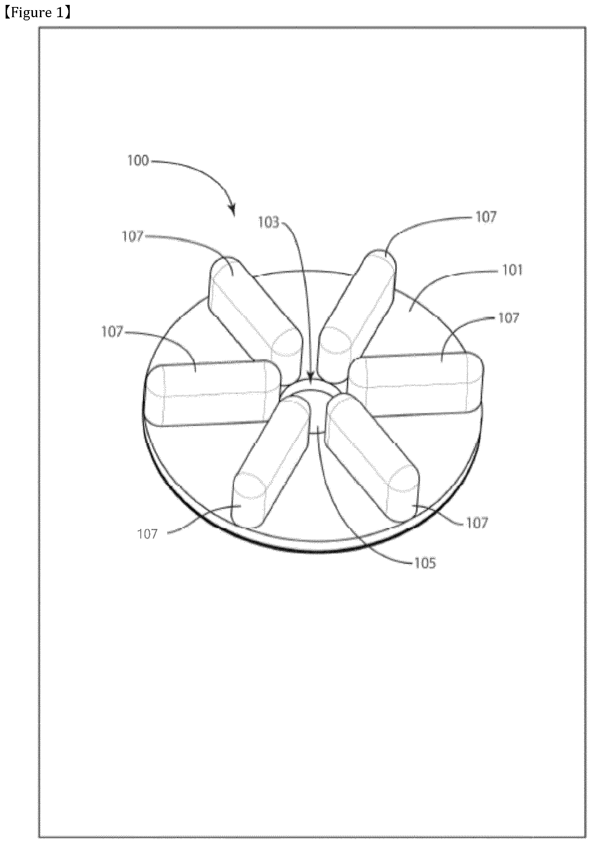

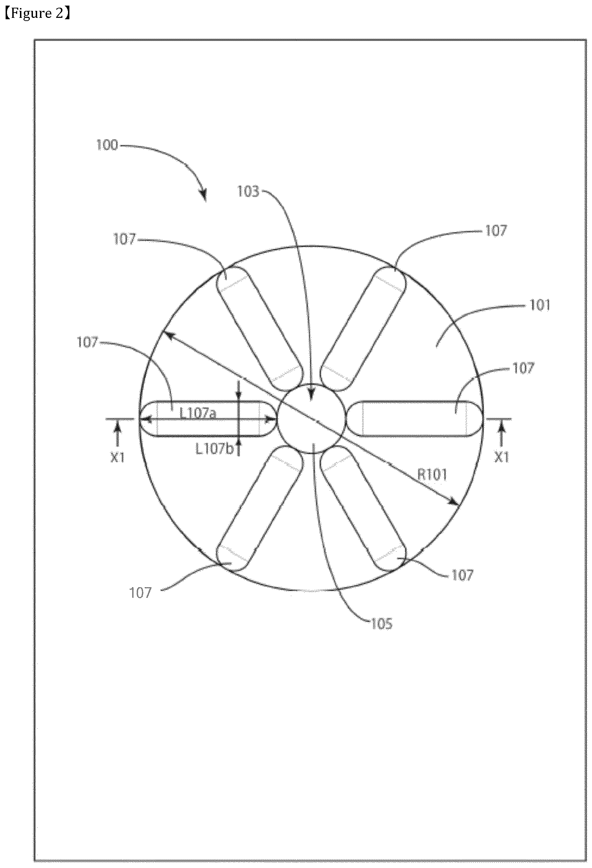

The configuration of the device 100 is described with reference to Fig. 1 to 3. Fig. 1, Fig. 2 and Fig. 3 are a perspective view of the device 100, a plan view thereof, and an X1-X1 cross-section of Fig. 2, respectively.

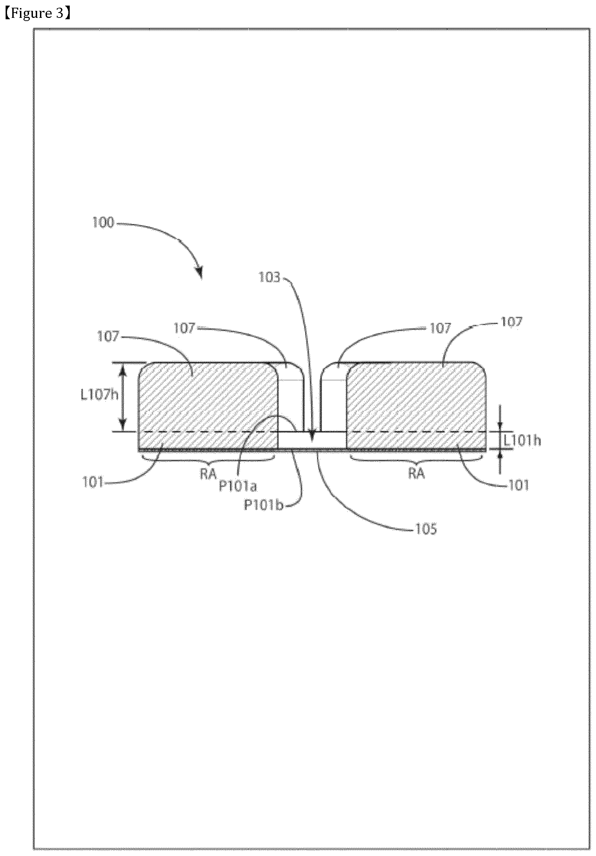

As shown in Fig. 1, the device 100 comprises a base 101, a through hole 103, a cell scaffold 105, and a manipulating part 107. The base 101 has a shape of a flat disk as shown in Fig. 2, and has an upper surface P101a and a lower surface P101b as shown in Fig. 3. The base 101 is made of a resin, such as polycarbonate, of which the specific gravity is larger than that of the medium. Because the device 100 including the base 101 needs to be located in the medium, the specific gravity of the base 101 and the like is determined to be, for example, 1.05 to 1.1 or more, by taking into account the specific gravity of the device 100 in relation to the medium and the buoyancy force generated in the device 100 in relation to the medium. As for the device 100 matching to the well W1 of a 96 well plate, the size of the base 101 is approximately 6 mm in diameter R101 (see Fig. 2) and approximately 0.5 mm in thickness L101h (see Fig. 3).

The through hole 103 is formed from the upper surface P101a to the lower surface P101b of the base 101 so as to penetrate the base 101. The through hole 103 is formed as a cylindrical shape, concentrically with the base 101. As for the device 100 matching to the well W1 of the 6 well plate, the diameter of the through hole 103 is approximately 1 mm.

The cell scaffold 105 is formed by a fiber sheet made of a polymeric material. The said polymeric material can be any materials as long as it does not exhibit cytotoxicity when the cells are cultured in contact with the said fiber sheet, and can be biodegradable or non-biodegradable, depending on the intended use of the cell sheet obtained by culturing the cells in contact with the fiber sheet Examples of biodegradable polymer materials include, for example, copolymers of polylactic acid and polyglycolic acid (PLGA), polyglycolic acid (PGA), polylactic acid (PLA), polyvinyl alcohol (PVA), polyethylene glycol (PEG), polyethylene vinyl acetate (PEVA), and polyethylene oxide (PEO), but are not limited to these. PLGA is preferably used because it is a safe material known to be hydrolyzed in vivo into lactic acids and glycolic acids originally present in living organisms, which are decomposed into water and carbon dioxide and then discharged from the body. By changing the ratio of combination ratio of PLA (polylactic acid) and PGA (polyglycolic acid), it is possible to adjust the rate of biodegradation of PGLA in vivo.

Examples of non-biodegradable polymer material include, for example, polystyrene (PS), polycarbonate (PC), polymethyl methacrylate (PMMA), polyvinyl chloride, polyethylene terephthalate (PET), polyamide (PA), and polymethyl glutarimide (PMGI), but are not limited to these. Polystyrene (PS), a material with low cytotoxicity, is particularly suitable for use. The cell scaffold 105 has a thin disc shape and is positioned along the lower surface P101b of the base 101.

As shown in Fig. 3, the cell scaffold 105 is disposed on the lower surface P101b of the base 101 by applying an adhesive to an adhesive region RA of the lower surface P101b of the base 101 and then crimping them together. The adhesive is applied to the lower surface P101b of the base 101 so as not to protrude from the through hole 103 when the cell scaffold 105 is disposed on the lower surface P101b of the base 101. At the stage of crimping the cell scaffold 105 onto the lower surface P101b of the base 101, the adhesive is absorbed into the gap of the cell scaffold 105 comprising a fiber sheet, and become integrated with the cell scaffold 105. For example, KE45 can be used as the adhesive.

Biomaterials such as cardiac myocytes and nerve cells and the like can be cultured three-dimensionally and retained in the through hole 103 of the cell scaffold 105. The biomaterial refers to (1) one or more cells or cell types, (2) one or more tissues or tissue types, or (3) organs or a part thereof. Examples of the cells include, for example, muscle cells such as cardiac myocytes and smooth muscle cells, hepatocytes as parenchymal cells of the liver, Kupffer cells, endothelial cells such as vascular endothelial cells and corneal endothelial cells, fibroblasts, osteoblasts, osteoclasts, periodontal ligament-derived cells, epidermal cells such as epidermal keratinocytes, epithelial cells such as tracheal epithelial cells, gastrointestinal epithelial cells, cervical epithelial cells, and corneal epithelial cells, mammary glandular cells, pericytes, renal cells, knees Langerhans islet cells, nerve cells such as peripheral nerve cells and optic nerve cells, chondrocytes, and bone cells. Examples of cardiac myocytes include pluripotent stem cells such as cells derived from ES cells or iPS cells. Examples of nerve cells include peripheral nerve cells and optic nerve cells. For retaining the cells on the cell scaffold 105, a cell suspension is dropped through the through hole 103 onto the cell scaffold 105, which is exposed from the through hole 103, using a pipette or the like, and then the cells are subjected to the cell culture.

As shown in Fig. 1, the manipulating part 107 is formed so that it protrudes from the upper surface P101a (see Fig. 3) of the base 101. The manipulating part 107 is formed in a plurality and radially on the upper surface P101a of the base 101, with the through hole 103 as the center. Specifically, six manipulating parts 107 are arranged on the upper surface P101a of the base 101 so that the angle between the adjacent manipulating part 107 is 60 degrees. Each manipulating part 107 has an upper surface arc shape, and a rectangular shape with a semicircle in the lateral direction in the cross-section along the upper surface P101a of the base 101. Similar to the base 101, the manipulating part 107 is made of a resin material such as polycarbonate, and is formed integrally with the base 101. The size of each manipulating part 107 is approximately 2.5 mm in the length in the longitudinal direction L107a (see Fig. 2), approximately 0.5 mm in the length in the lateral direction L107b (see Fig. 2), and approximately 1.3 mm in the height L107h (see Fig. 3).

By forming a plurality of the manipulating part 107 radially, the user can easily hold and manipulate the manipulating part 107 with tweezers or the like, simply by adjusting the angle of the wrist, regardless of the positional relationship between the user and the manipulating part 107 when placing the device 100 in the well W1. By forming six manipulating part 107, the user can easily manipulate the manipulating part 107.

### 2. Method of Use

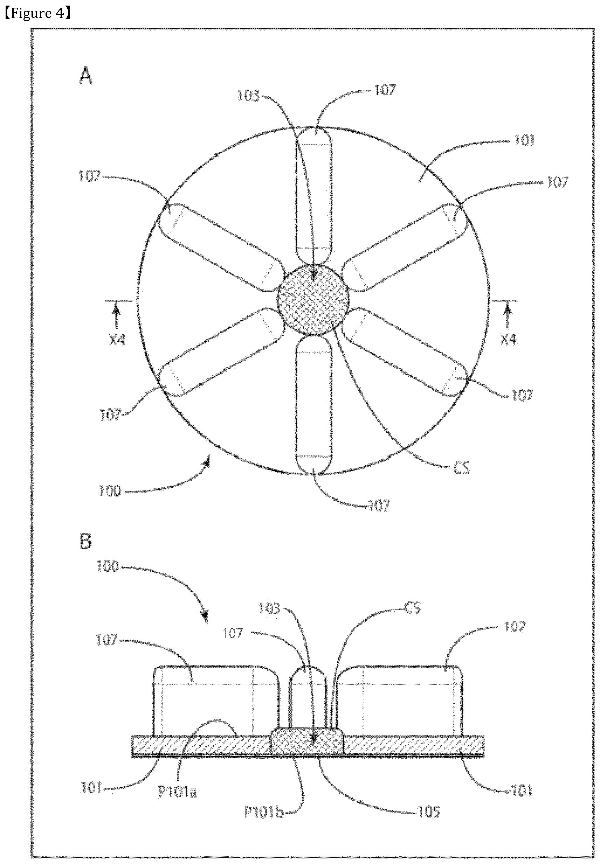

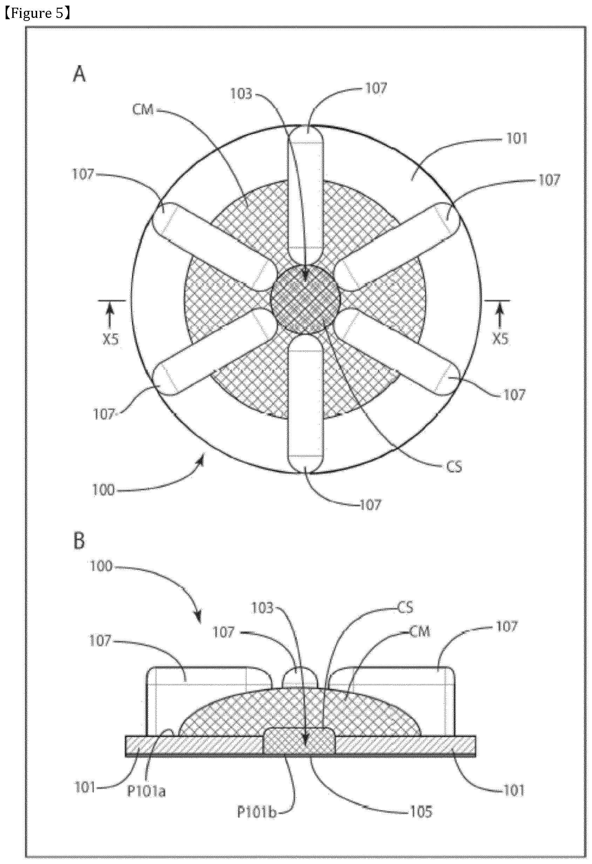

Fig, 4 and Fig. 5 are used to explain how to use the device 100, divided into two sections: when cells are cultured and retained in the device 100, and when the device 100 retaining the cells are manipulated. In Fig. 4, A shows a plan view of the device 100 where the suspension CS is stored, and B shows an X4-X4 cross-section of Fig. 4-A. In Fig. 5, A shows a plan view of the device 100 where the medium is stored, and B shows an X5-X5 cross-section of Fig. 5-A.

### (1) When culturing and retaining cells on the device 100

In the device 100, when the cells are cultured and retained on the cell scaffold 105 exposed through the through hole 103 of the base 101, cell suspension is dropped into the inside of the through hole 103 using a pipette or the like. This allows the cell suspension to be stored inside the through hole 103 as shown in Fig. 4-A. The surface tension of the cell suspension allows the cell suspension to be stored in the inside of the through hole 103 until the shape of the cell suspension becomes convex from the upper surface P101a of the base 101 upwardly without flowing out of the through hole 103, as shown in FIG. 4B. The cell suspension can be stored in the through hole 103 until it projects from the upper surface P101a of the base 101 but without flowing over out of the through hole 103 due to the surface tension of the suspension. If the base 101 is made of a hydrophobic material, the cell suspension can be more stably stored inside the through hole 103.

After storing the cell suspension inside the through hole 103, allowing the cells to settle and be retained on the cell scaffold 104 for a few hours to one day, a pipette or the like is used to store the medium on the upper surface P101a of the base 101 (see Fig. 5-B) as shown in Fig. 5-A. Namely, a part of the space above the upper surface P101a of the base 101 functions as a medium storage space, and the other part of the upper surface P101a of the base 101 functions as a space for forming the medium storage space.

As shown in Fig. 5-B, the surface tension allows the medium to be stored in a convex shape upwardly within the upper surface P101a of the base 101. When the base 101 is made of a hydrophobic material, the medium can be stored more stably convexly on the upper surface P101a of the base 101. The amount of the medium to be added is, for example, approximately 20 µl in the case of culturing cardiac myocytes in the device 100 having a diameter R101 of the base 101 is approximately 6 mm (see Fig. 2), which fits to the well W1 of a 96 multi-well plate.

As described above, by storing additional medium on the base, it is possible to provide fresh medium for the cells that are retained, thus allowing the cells to be stably cultured for a longer period.

### (2) When manipulating the device 100

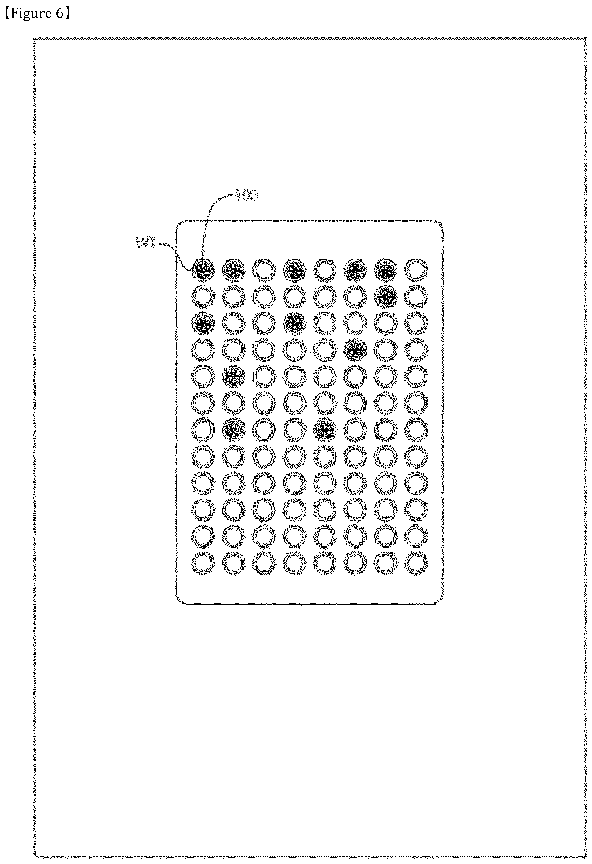

When manipulating the device for retaining and transporting cells 100, the manipulating part 107 is held by a holding tool such as tweezers or the like. This allows the device 100 to be easily manipulated. For example, the device 100 is transferred from outside the well W1 of the multi-well plate to inside the well1, as shown in Fig. 6, by holding the manipulating part 107 with a holding tool such as tweezers or the like. Similarly, the position and orientation of the device 100 is changed within the well W1. In particular, even with a small cell device 100 that fits for each well W1 of a multi-well plate having ninety-six well W1, the device 100 can be easily manipulated with the manipulating part 107. When transporting the device 100, it is possible to manipulate the device 100 by holding the base 101 with a holding tool.

In the device 100 where the cells are cultured and retained and additional medium is arranged, the spillage of the medium can be prevented by holding the manipulating part 107 protruding from the medium with a holding tool.

After performing various experiments, such as pharmacology experiments, in the well W1 of a 96 multi-well plate with the device 100 in place, the device 100 can be easily transferred by holding the manipulating part 107 with a holding tool onto, for example, the electrode of an MEA (Multi-Electrode Array) probe for the measurement of the electrical characteristics of the cells.

### [Example 2]

In Example 1, the medium was placed on the upper surface P101a of the base 101 by utilizing the surface tension of the medium and the hydrophobicity of the base 101 and the manipulating part 107. On the other hand, the device 200, an example of the device of the present invention, has a medium storage space forming part 209 that forms a medium storage space S209 for storing the medium above the through hole 103, which facilitates addition of the medium on the upper surface P101a of the base 101. Hereinafter, in the case of the similar configurations to Example 1 are marked with the same signs, and detailed descriptions are omitted.

### 1. Configuration

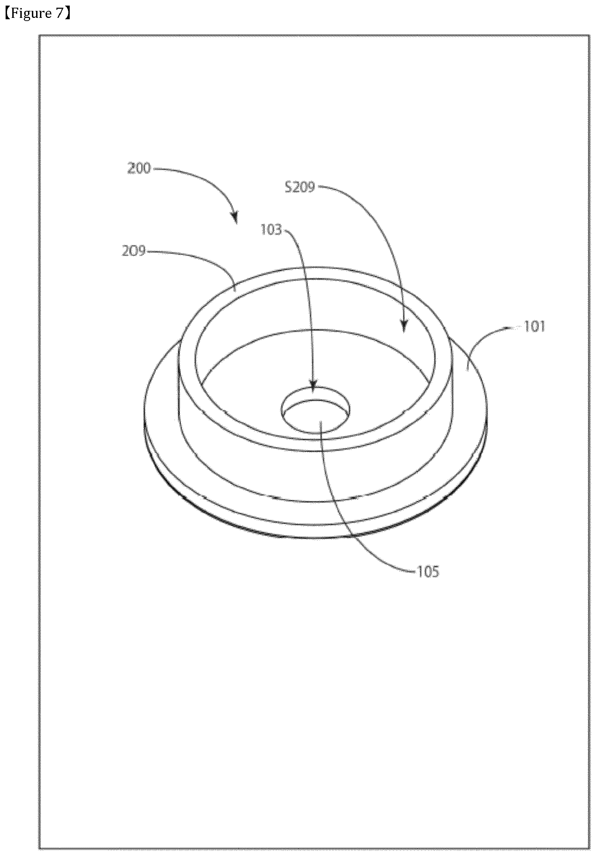

The configuration of the device 200 is described here with reference to Fig. 7. Fig. 7 is a perspective view of the device 200.

As shown in Fig. 7, the device 200 comprises a base 101, a through hole 103, a cell scaffold 105, and a medium storage space forming part 209.

The medium storage space forming part 209 is formed so as to project from the upper surface P101a of the base 101 (see Fig. 3). The medium storage space forming part 209 is formed in a cylindrical shape on the upper surface P101a of the base 101 with the through hole 103 at its center. The medium storage space forming part 209 is made of a resin material similar to the base 101, and is formed in an integrated manner with the base 101.

By forming the medium storage space forming part 209, a medium storage space S209 can be formed above the through hole 103. The size of the medium storage space forming part 209 is approximately 4.4 mm in the inner diameter, approximately 5.4 mm in the outer diameter, and approximately 1.3 mm in the height in the case of the device 200 matching to the well W1 of the 96 multi-well plate.

The medium storage space forming part 209 is formed so as to project from the upper surface P101a of the base 101, and therefore also functions as a manipulating part.

### 2. Method of Use

Fig. 7 is used to explain how to use the device 200, divided into two sections: when cells are cultured and retained in the device 200, and when the device 200 retaining the cells are manipulated. In Fig. 7, A is a perspective view of the device 200 where the suspension CS is stored.

### (1) When culturing and retaining cells on the device 200

The procedure of culturing and retaining cells on the cell scaffold 105 in the device 200 is similar to that for the device 100.

After storing the cell suspension inside the through hole 103, allowing the cells to settle and be retained on the cell scaffold 105 for a few hours to one day, a pipette or the like is used to store the medium in the medium storage space S209 formed by the medium storage space forming part 209 that is located on the upper surface P101a of the base 101.

In the device 100 described in Example 1, the medium was stored in a convex shape upwardly within the upper surface P101a of the base 101 due to the surface tension of the added medium and/or the hydrophobicity of the base 101 (see Fig. 5-B), and therefore it may be difficult to stably store the medium. On the other hand, the medium storage space S209 is formed by the medium storage space forming part 209 in the device 200, which enables stable storage of the medium in the medium storage space S209 on the upper surface P101a of the base 101.

As described above, by storing an additional medium on the base, it is possible to 101 to provide a fresh medium to held cells, so that the cells can be stably cultured for a longer period.

### (2) When manipulating the device for retaining and transporting cells 200

When manipulating the device 200, the medium storage space forming part 209 and the base 101 is held by a holding tool such as tweezers or the like. This allows the device 200 to be easily manipulated.

### [Example 3]

In Example 2, the device for retaining and transporting cells 200 had a medium storage space formingpart209 formed in a cylindrical shape. On the other hand, the device 300, an example of the device of the present invention, has a plurality of medium storage space forming part 209. Hereinafter, in the case of the similar configurations to Example 1 and Example 2 are marked with the same signs, and detailed descriptions are omitted.

### 1. Configuration

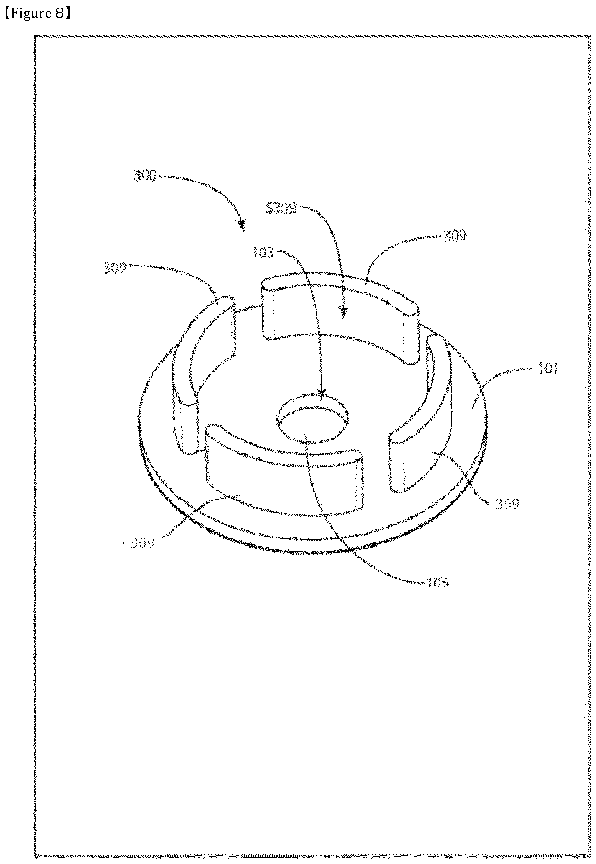

The configuration of the device 300 is described here with reference to Fig. 8. Fig. 8 is a perspective view of the device 300.

As shown in Fig. 8, the device 300 comprises a base 101, a through hole 103, a cell scaffold 105, and a medium storage space forming part 309.

A plurality of medium storage space forming part 309 is formed so as to project from the upper surface P101a of the base 101 (see Fig. 3). The medium storage space forming part 309 is formed independently of the other adjacent medium storage space forming part 309 with a gap. A plurality of medium storage space forming part 309 forms a cylindrical shape centered on the through hole 103 on the upper surface P101a of the base 101, and the medium storage space S309 inside the medium storage space formation part 309. The medium storage space forming part 309 is made of polycarbonate similar to the base 101, and is formed in an integrated manner with the base 101.

The medium storage space forming part 309 is formed so as to project from the upper surface P101a of the base 101, and therefore also functions as a manipulating part.

### 2. Method of Use

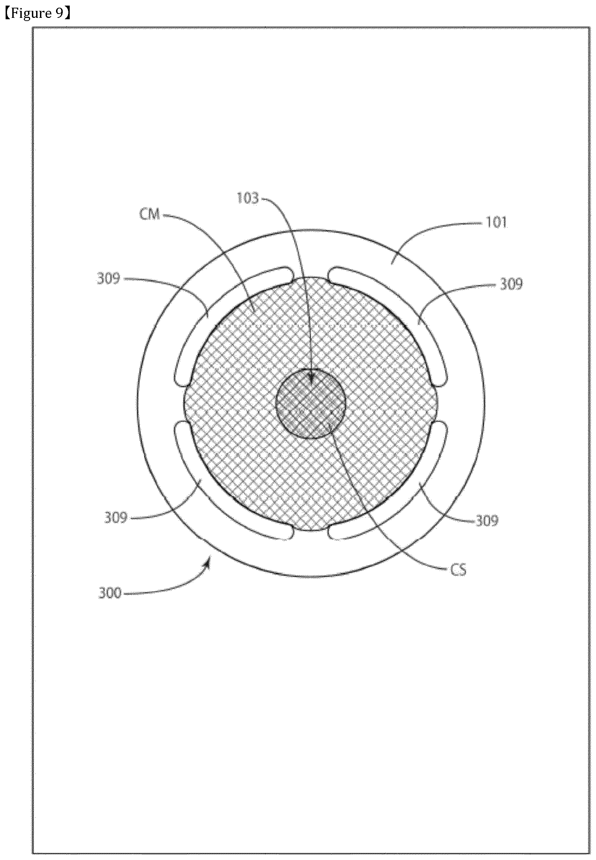

Fig.9 is used to explain how to use the device 300, divided into two sections: when cells are cultured and retained in the device 200, and when the device 200 retaining the cells are manipulated. Fig. 9 shows a plan view of the device 300 where the suspension CS and the medium are stored.

### (1) When culturing and retaining cells on the device 300

The procedure of culturing and retaining cells on the cell scaffold 105 in the device 300 is similar to that for the device 100. Also, the procedure of storing medium in the medium storage space S309 formed by the medium storage space forming part 309 in the device 300 is similar to that for the device 200.

However, the medium storage space S309 is formed by a plurality of medium storage space forming parts 309. Namely, it means that the medium storage space S309 will have an opening leading to the outside of the medium storage space formation portion 309, but as shown in Fig. 9, the surface tension of the medium to be added and/or the hydrophobic nature of the base portion 101 allows the medium to be stably stored in the medium storage space S309 (see above, Fig. 8).

As described above, by storing an additional medium on the base, it is possible to 101 to provide a fresh medium to held cells, so that the cells can be stably cultured for a longer period.

### (2) When manipulating the device for retaining and transporting cells 300

When manipulating the device 300, the medium storage space forming part 309 and the base 101 is held by a holding tool such as tweezer or the like. This allows the device 200 to be easily manipulated.

### [Example 4]

In Example 1, when placing the device 100 in the well W1, it is necessary to submerge the device 100 in the medium stored in the well W1. However, if there is not much difference between the inner diameter of the well W1 and the diameter R101 (see Fig. 2) of the base 101 of the device 100, the medium may enter into the gap between the side surface of the well W1 and the device 100, and the air entering under the device 100 may not escape outside from the well W1 and the device 100. On the other hand, the device 400, which is an example of the device for retaining and transporting cells of the present invention, has the base 101 and an air vent 413 that penetrates the cell scaffold 105 located at the bottom of the base 101, which allows the air that has entered under the device 400 to be easily discharged outside. Hereinafter, the same configuration as in Example 1 are marked with the same sign and detailed description are omitted.

### 1. Configuration

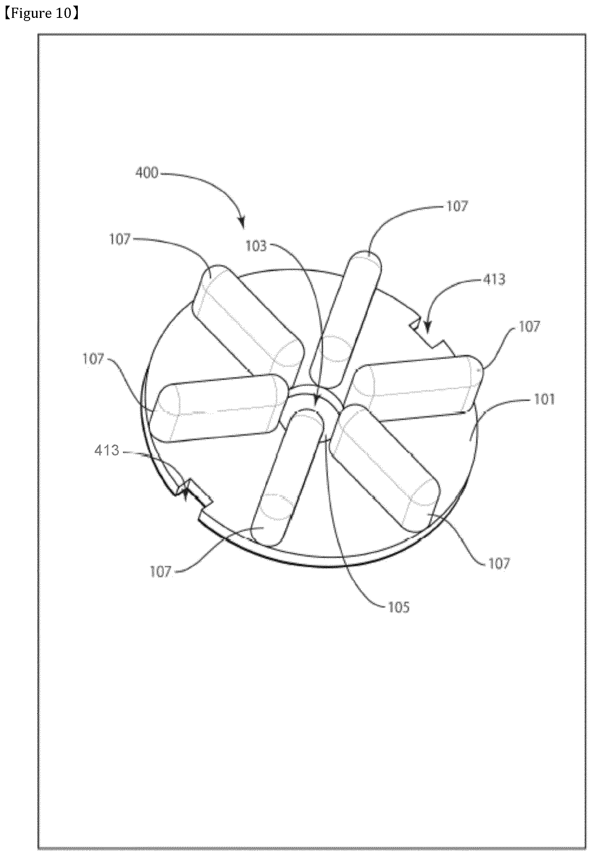

The configuration of the device 400 is described here with reference to Fig. 10. Fig. 10 is a perspective view of the device 400.

As shown in Fig. 10, the device 400 comprises a base 101, a through hole 103, a cell scaffold 105, a manipulating part 107, and an air vent 413.

The air vent 413 is formed as a rectangular notch from the outer periphery of the base 101 towards the inside, and is formed so as to penetrate the base 101 and the cell scaffold 105. It enables easy discharge of the air that entered below the device 400 from between the base 101 and the side surface of the well W1.

### 2. Method of Use

The method of using the device 400 is similar to that the device 100. However, it is necessary to immerse the device 400 in the medium in the well W1 while confirming that the air escapes from the air vent 413.

### [Example 5]

The device for retaining and transporting cells 1700 of the present invention has a simple structure for easier use in which cells can be easily cultured. Hereinafter, in the case of the similar configurations to Example 1 are marked with the same signs, and detailed descriptions are omitted.

### 1. Configuration

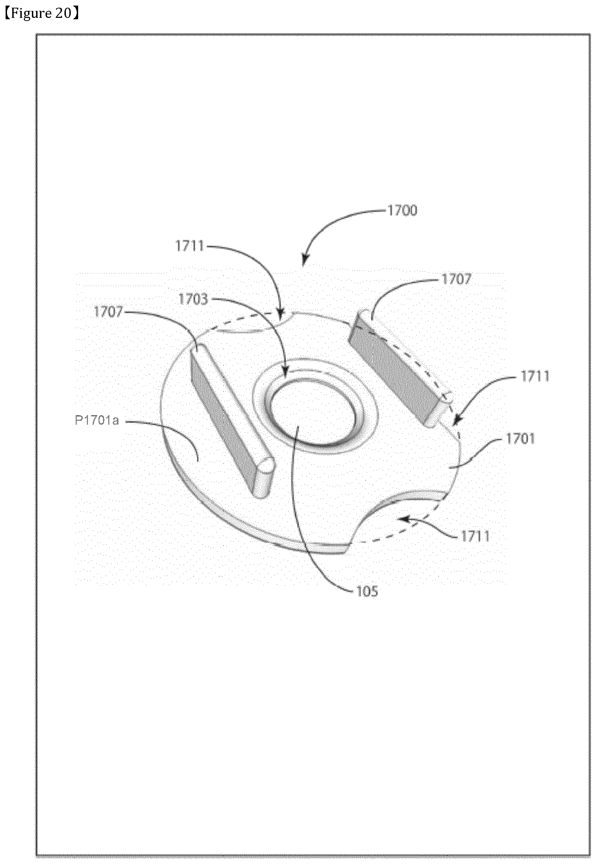

The configuration of the device 1700 is described here with reference to Fig. 20. Fig. 20 is a perspective view of the device 1700.

As shown in Fig. 20, the device 1700 has a base 1701, a through hole 1703, a cell culture holding part 105, a manipulating part 1707, and an air vent 1711.

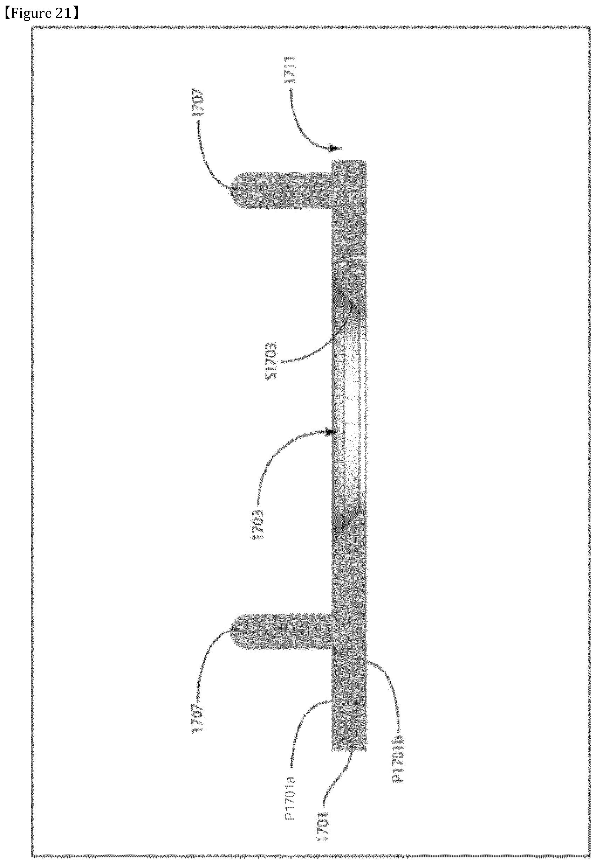

The base 1701 has a thin disc shape and is made of, for example, polycarbonate. The through hole 1703 is formed in the center of the base 1701. The base 1701 also has an upper surface P1701a and a lower surface P1701b (see Fig. 21).

The through hole 1703 is formed so as to penetrate the base 1701 from the upper surface P1701a to the lower surface P1701bof the base 1701. The through hole 1703 has a shape of a circular truncated cone that narrows from the upper surface P1701a where the manipulating part 1707 is formed towards the lower surface P1701b. Therefore, the opening on the upper surface P1701a of the base 1701 can be enlarged, which enables easy dropping of the suspension into the through hole 1703. Furthermore, since the capacity of the through hole 1703 can be increased, a larger volume of the suspension can be retained in the through hole 1703.

The shape of the through hole 1703 is described here with reference to Fig. 21. In Fig. 21, A shows the axial cross-section of the base 1701 and the manipulating part 1707. The through hole 1703 has an inclined part S1703 at a prescribed distance from the lower surface P1701b of the base 1701 towards the upper surface P1701a. The inclined part S1703 is formed as a surface inclined by a prescribed angle from the axial direction. The axial cross-section of the inclined part S1703 is an inclined straight line. By forming the inclined part S1703 in the through hole 1703, the yield of cell seeding during cell culture can be improved.

Returning to Fig. 20, the manipulating part 1707 is formed so as to project from the upper surface P1701a of the base 1701. The manipulating part 1707 is formed in parallel on the upper surface P1701a of the base 1701 across the through hole 1703. As a result, the manipulating part 1707 can be formed apart from the through hole 1703, so that the culture solution stored in the through hole 1703 can be prevented from exceeding its own surface tension and overflowing from the through hole 1703 when culturing cells.

Since the length of the manipulating part 1707 can be made longer compared to when forming the manipulating part 1707 radially centered on the through hole 1703, the device 1700 can be easily manipulated by holding with tweezers etc.

The manipulating part 1707 is made of a resin material such as polycarbonate as the base 1701, and is formed integrally manner with the base 1701. The manipulating part 1707 allows the user to hold it with tweezers or the like, and to easily manipulate the device 1700.

The air vent 1711 is formed as an area in which a part of the outer peripheral part of the base 1701 is cut off. The cell culture holding part 1701 located below the base 1701 is also formed in the same shape as the base 1701. It enables easy discharge of the air that has entered below the device 1700 when putting the device 1700 in a cell culture container such as one well of a multi-well plate.

### 2. Method of Use

The method of using the device 1700 is similar to that the device 100 - 400.

The method of using the device for retaining and transporting cells 1700 when measuring cell characteristics is described here by taking an example of measuring an extracellular potential generated by cell activity of cardiac myocytes that is formed in the device for retaining and transporting cells 1700.

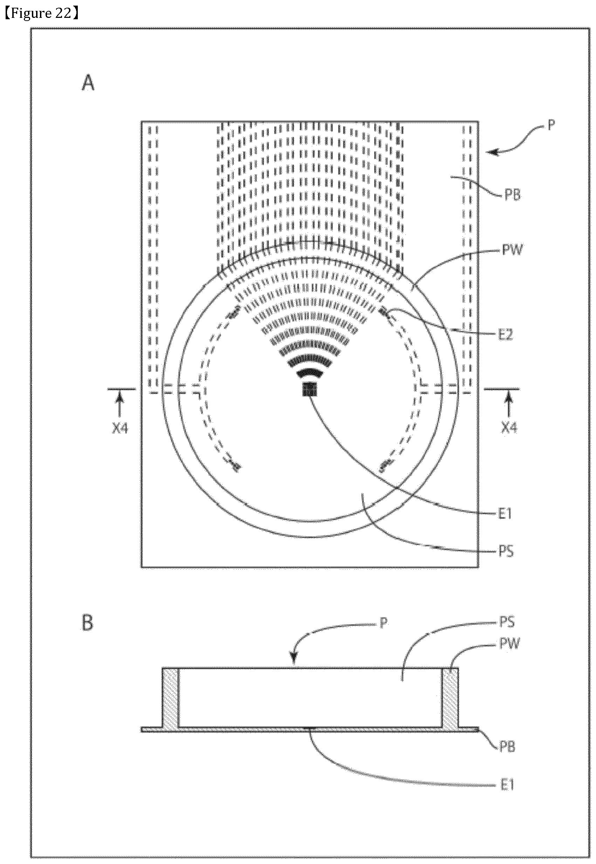

An MEA (Multi-Electrode Array) probe P is used for measuring the extracellular potential. The MEA probe P is a device for measuring the extracellular potential generated by cellular activity. Fig. 22 shows a brief description of the MEA probe P. Fig. 21-A is a plan view of the MEA probe P, and Fig. 22-B is an X4-X4 cross-section of the MEA probe P shown in Fig. 22-A. As shown in Fig. 22-A and Fig. 22-B, the MEA probe P has a substrate PB and a molten wall PW. The substrate PB has a thin plate shape, and the molten wall PW is formed on the substrate PB. The melted wall PW has a cylindrical shape with one end open, and stores a medium inside, forming a cell arrangement space PS for placing the cells to be tested.

The MEA probe P has also measurement electrodes E1 and reference electrodes E2. The measurement electrodes E1 are formed along with the substrate PB at approximately the center of the molten wall PW. The measurement electrodes E1 is arranged in a 4×4 matrix. A prescribed number of the reference electrodes E2 are arranged around the measurement electrodes E1. The MEA probe P measures the extracellular potential associated with the cellular activities based on the potential difference between the measurement electrodes E1 and the reference electrodes E2. A lead line (dotted line in the figure) is arranged from each of the measurement electrodes E1 and the reference electrodes E2.

After adding a predetermined amount of the medium into the cell arrangement space PS of the MEA probe P, the device 1700 where cardiac myocytes are cultured is placed in the cell arrangement space PS. At this time, the device 1700 is arranged so that the regions corresponding to the through hole 1703 in the cell culture holding part 105 is located on the measurement electrodes E1 of the MEA probe P.

The MEA probe P is then used to measure the extracellular potential associated with the cellular activities of the cardiac myocytes cultured in the device 1700.

### 3. Example of experiment

The extracellular potential of the cultured cardiac myocytes was measured using the device 1700 that is formed using polycarbonate and that has a base 1701 (diameter: 6 mm, thickness: 0.7 mm), a through hole 1703 (diameter: 1.5 mm), a cell culture holding part 105 made of an oriented fiber sheet with a pitch of 10 µm, a manipulating part 1707, and the air vent section 1711, and the MEA probe P (MED64 System, Alpha MED Scientific Inc.). The oriented fiber sheet of the cell culture holding part 105 was seeded with human iPS cell-derived cardiac myocytes, and the cardiac myocytes were cultured for seven days at 37°C with 5% CO₂. The pitch of the fiber sheet refers to the distance between the core lines of adjacent fibers among the fibers composing of the fiber sheet.

The drug response of the cultured cardiac myocytes can be measured by means of the extracellular potential by changing the conditions of the drug to be administered to the cultured cardiac myocytes. Dofetilide (a drug for atrial fibrillation, Sigma) was chosen as the drug to be administered.

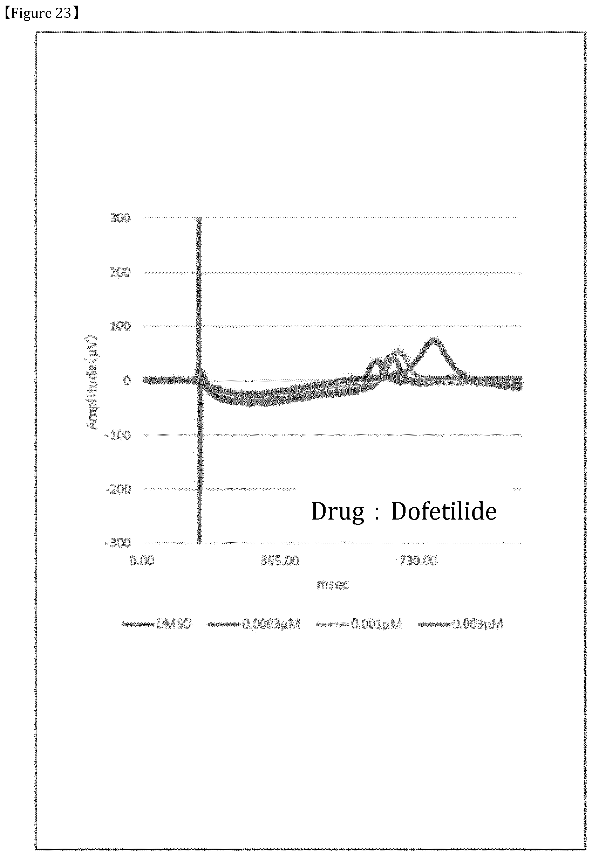

The device 1700 where the cardiac myocytes were cultured was placed in the cell arrangement space PS of the MEA probe P filled with medium. Then the extracellular potentials of each dose: DMSO (Dimethyl Sulfoxide) alone; dofetilide: 0.0003 µM; dofetilide: 0.001 µM; and dofetilide: 0.003 µM. The result is shown in Fig. 23.

The results in Fig. 23 show that the first and the second peak potentials caused by the beating of the cardiac myocytes change depending on the dose. Specifically, as the concentration of the dofetilide increases, the time required from the first peak potential to the second peak potential becomes longer. It means that the time between the first peak and the second peak is prolonged depending on the concentration. Therefore, the drug response as a cellular characteristic can be examined using the device 1700.

### [Other Embodiments]

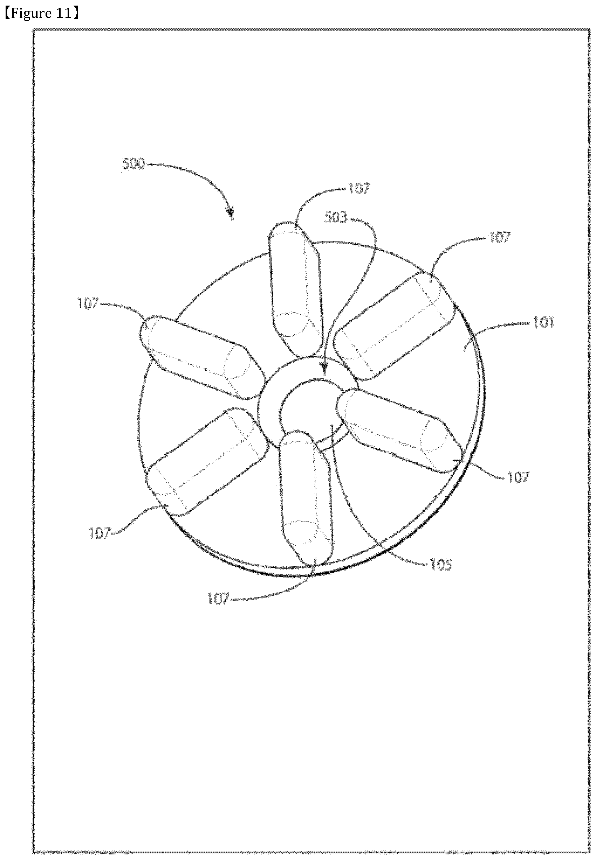

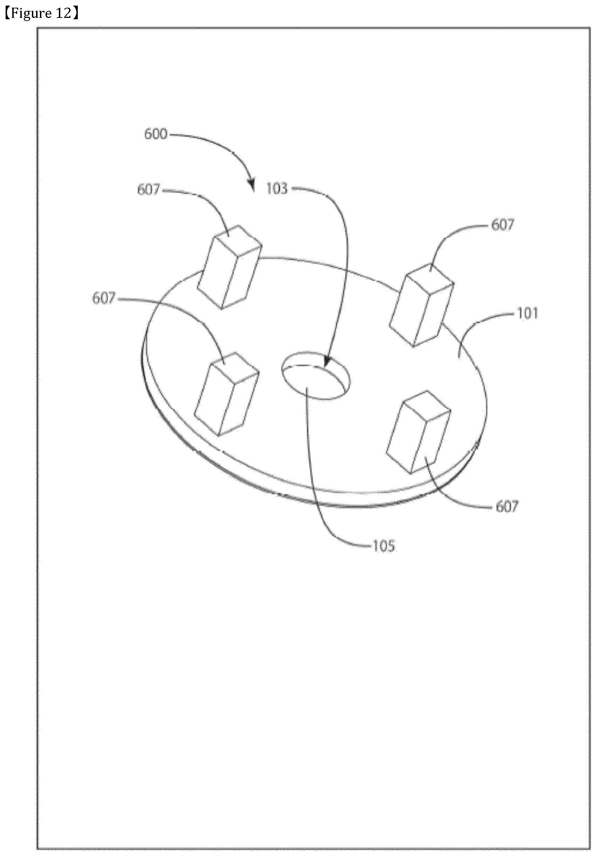

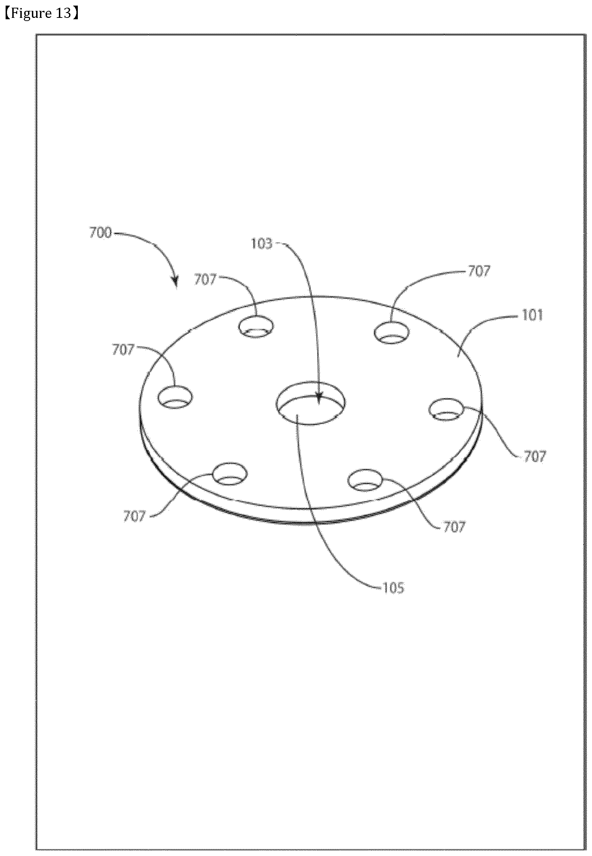

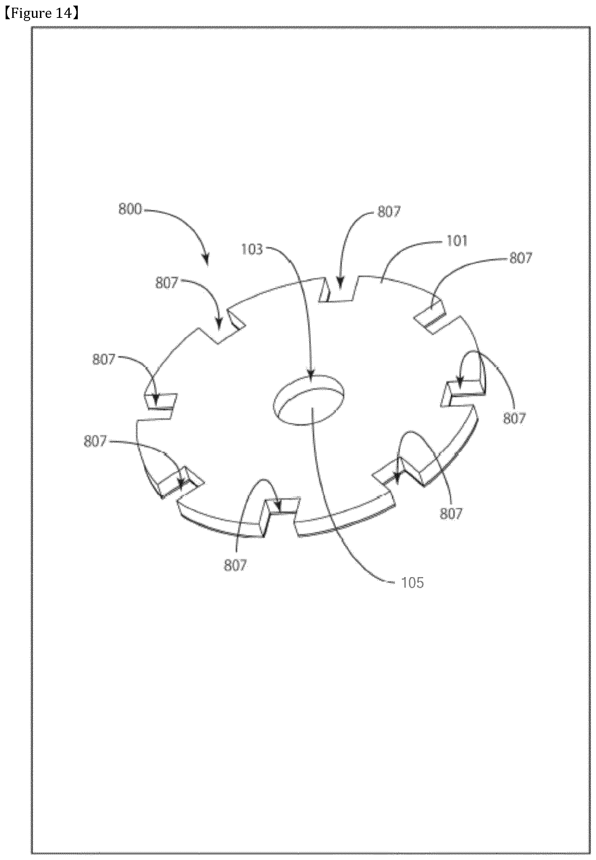

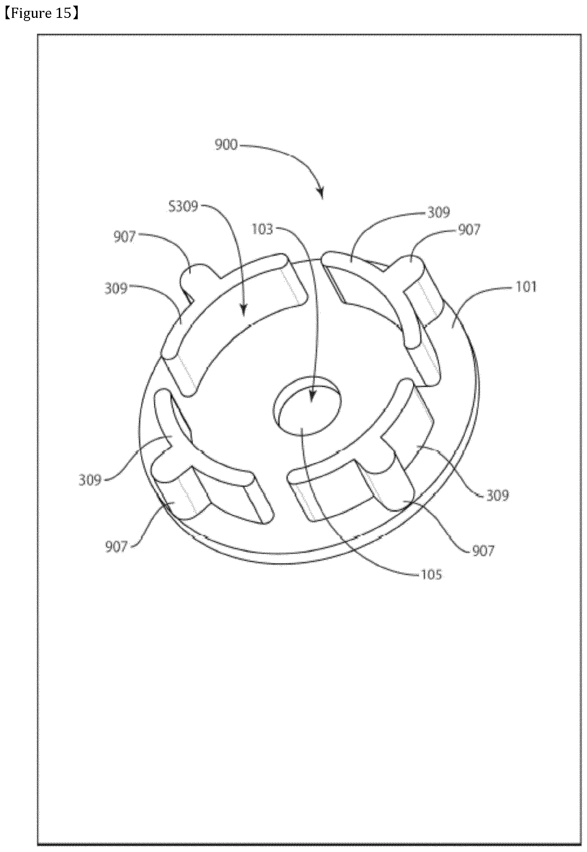

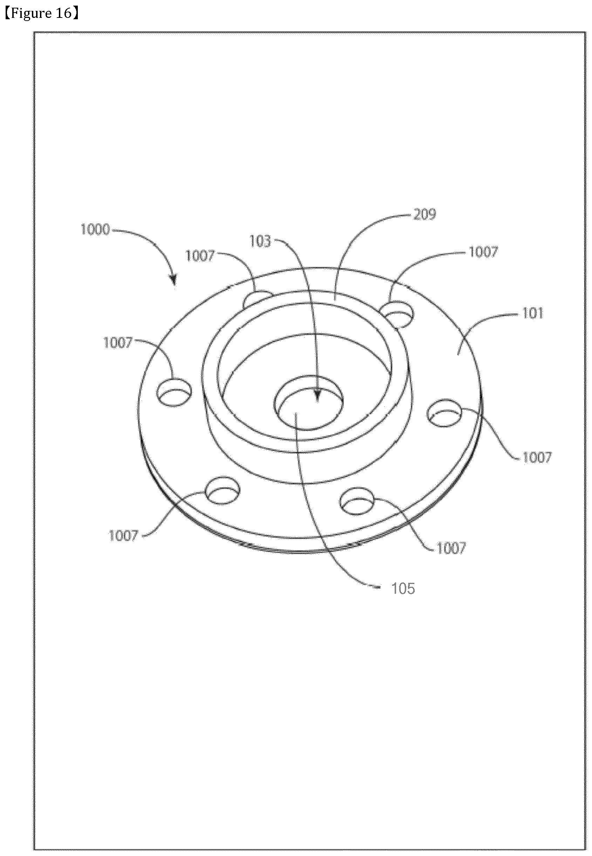

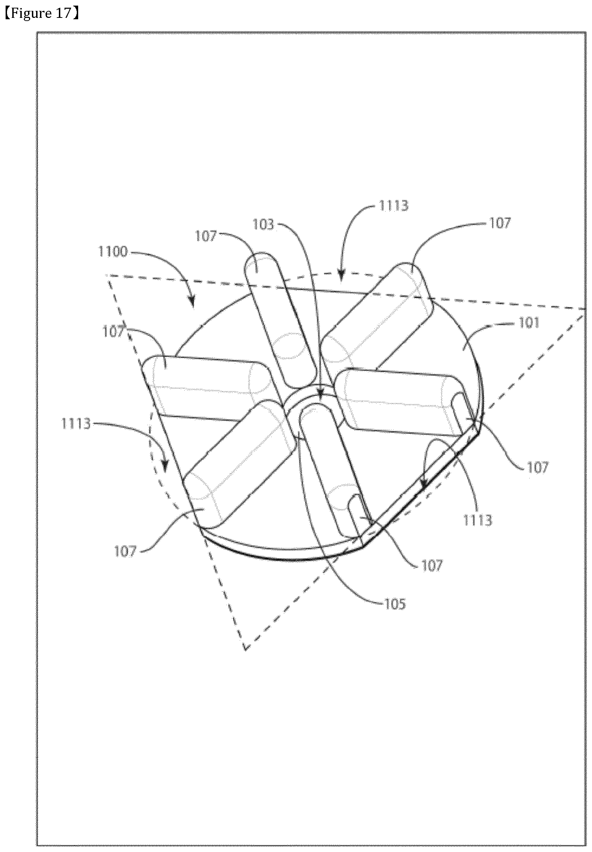

(1) The shape of the through hole 103: In Example 1, the through hole 103 has a cylindrical shape penetrating through the upper surface P101a and the lower surface P101b of the base 101, but the shape is not limited to the example as long as itpenetrates through the upper surface P101a and the lower surface P101b. For example, the through hole 503 may have a shape of a circular truncated cone that narrows from the upper surface P1701a where the manipulating part 1707 is formed towards the lower surface P1701b (see Fig. 3), similarly to the device for retaining and transporting cells 500 shown in Fig. 11. Therefore, the opening on the upper surface P101a of the base 101 can be enlarged, which enables easy dropping of the suspension into the through hole 503. Furthermore, since the capacity of the through hole 503 can be increased, a larger volume of the suspension can be retained in the through hole 503. The same applies to Example 2 and Example 3.
(2) The shape of the manipulating part 107: As for the device 100 in Example 1, multiple manipulating part 107 is formed radially, but the shape is not limited to the example as long as it protrudes from the upper surface P101a of the base 101. For example, multiple manipulating part 607 with a protruding pillar shape can be formed as the device for retaining and transporting cells 600 shown in Fig. 12.
   Furthermore, it is not limited to the example as long as it can be held by a holder and can manipulate the device for retaining and transporting cells. For example, it can form the manipulating part 707 of the through hole or the like that is formed in a concave shape from the upper surface P101a of the base 101 (see Fig. 3) similarly to the device 700 shown in Fig. 13. As the device for retaining and transporting cells 800 shown in Fig. 14, it can also form multiple notch-shaped operating unit 807 on the outer peripheral surface of the base 101 in a gear shape.
(3) Medium storage space forming part 209: As for the device 200 in Example 2, the medium storage space forming part 209 is an integrated unit that also function as a manipulating part. However, the manipulating part 907 protruding from the upper surface P101a of the base 101 can be installed on the device 300 in Example 3, similarly to the device shown in Fig. 15. Furthermore, the manipulating part 1007 formed in a concave shape from the upper surface P101a of the base 101 can be installed on the device 200 in Example 2, as the device 1000 shown in Fig. 16. The same applies to Example 3 and others.
(4) Air vent 413: In the device 400 of Example 4, the air vent 413 was formed as a rectangular notch from the outer periphery of the base 101 towards the inside, but it is not limited to the example as long as it can discharge the air entered below the device. For example, it may form the air vent 1113 of which a part of the outer periphery of the base 101 and the cell scaffold 105 located thereunder has been cut off, similarly to the device 1100 shown in Fig. 17. The base 101, the cell scaffold 105, and in some cases the manipulating part 107 can be cut in a triangular shape as shown in Fig. 17, a polygonal shape such as a hexagon, or at any designated places.
(5) Cells to be cultured: In Example 1 to 4, cardiac myocytes and nerve cells were shown as the cells to be cultured, but the cells to be cultured are not limited to the examples. For example, other cells such as neural cells derived from pluripotent stem cells may be used. The pluripotent stem cells include, for example, embryonic stem cells (ES cells) and iPS cells.
(6) 96 multi-well plate: In Example 1 to 4, the device 100 was arranged in the well W1 of the 96 multi-well plate, but the vessel is not limited to limited to the examples as long as it can accommodate the device 100 and can be used for the cell culture. For example, culture dish with a single well can be used.
(7) Integrality between the base 101 and the manipulating part 107: In Example 1, the base 101 and the manipulating part 107 were formed integrally, but they may also be formed as separate bodies and adhered with an adhesive material. The same applies to other embodiments than Example 1.
(8) Placement in well W1: In Example 1, the device for retaining and transporting cells 100 was immersed in the medium stored in the well W1 when placing the device 100 in the well W1, but the method is not limited to the example as long as the device 100 can be installed in the well W1. For example, the medium may be poured from the space between the device for retaining and transporting cells 100 and the sidewall of the well W1 after placing the device 100 in the well W1.
(9) Cultured cells: In Example 1, the cells were cultured three-dimensionally on the cell scaffold 105 in the through hole 103, but they may also be cultured two-dimensionally.
(10) Measurement of cell characteristics: In Example 5, the drug response as cell characteristics was measured as an example of the use of the device 1700, but it is not limited to the cell characteristics. For example, patch clamp, imaging, biomarker, or the like can be used.
   Also, dofetilide was used as the drug for measuring the drug response, but it can also be other drug. The same applies to the other embodiments.
(11) Base 101: In Example 1, the base 101 had a flat plate shape, but it is not limited to the example as long as it has an upper and a lower surface, such as a cylindrical shape with the upper end closed or a pillar shape.

Furthermore, the base 101 can be formed by a metal, particularly that having low or no biotoxicity instead of a resin material. It enables adjustment of the weight of the device. The base 101 may be formed by combining a resin material and a metal, for example in the same shape, or by using a metal as a part of the resin material. The device for retaining and transporting cells 100 can also be formed by different components with different molding materials, such as using the resin-made base 101 and the metal-based manipulating part 107. Furthermore, the base 101 and the manipulating part 107 can be formed by combining separate components made of the same material. The same applies to the other embodiments.

### [Industrial Applicability]

The device for retaining and transporting cells of the present invention can be used, for example, for drug efficacy testing of cells using 96 multi-well plates.

### [Description of Symbols]

- 100: Devise for retaining and transporting cells
101 Base
P101a Upper surface
P101b Lower surface
103 Through hole
105 Cell scaffold
107 Manipulating part
- 200: Devise for retaining and transporting cells
209 Medium storage space forming part
S209 Medium storage space
- 300: Devise for retaining and transporting cells
309 Medium storage space forming part
S309 Medium storage space
- 400: Devise for retaining and transporting cells
413 Air vent
- 500: Devise for retaining and transporting cells
503 Through hole
- 600: Devise for retaining and transporting cells
607 Manipulating part
- 700: Devise for retaining and transporting cells
707 Manipulating part
- 800: Devise for retaining and transporting cells
807 Manipulating part
- 900: Devise for retaining and transporting cells
907 Manipulating part
- 1000: Devise for retaining and transporting cells
1007 Manipulating part
- 1100: Devise for retaining and transporting cells
1113 Air vent
- 1700: Devise for retaining and transporting cells
1701 Base
P1701a Upper surface
P1701b Lower surface
1703 Through hole
105 Cell scaffold
1707 Manipulating part
1711 Air vent

## Claims

1. A device arranged in a cell culture container for holding and transporting cells, which comprises a base with an upper and a lower surface;
a through hole formed so as to penetrate the said base;
a cell scaffold to hold the cells located along the lower surface of the said base; and
a manipulating part that can be held by a specified holding tool.

2. The device according to claim 1, which further comprises a medium storage space forming part that forms a medium storage space on the said through hole.

3. The device according to claim 1 or claim 2, wherein the said manipulating part is protruding from the upper surface of the said base.

4. The device according to claim 1 or claim 2, wherein the said manipulating part is formed concaving from the upper surface of the said base.

5. The device according to any one of claims 1 to 4, which further comprises an air vent penetrating the said base.

6. The according to any one of claims 1 to 5, wherein the said through hole has a shape narrowing from the surface of the said protrusion towards the opposite surface.

7. The device according to any one of claims 1 to 6, wherein the said base is formed as a flat plate.

8. The device according to any one of claims 1 to 7, wherein the said base is at least made of a material that has larger specific gravity than the medium.

9. The device according to any one of claims 1 to 8, of which the said cell scaffold is a thin fiber sheet.

10. The device according to claim 9, the said fiber sheet is prepared by a polymeric material.

11. The device according to any one of claims 1 to 10, wherein the said cell scaffold comprises biomaterials.

12. The device according to claim 11, wherein the said cell scaffold comprises specified cells.

13. The device according to claim 11, wherein the said cell scaffold comprises cardiac myocytes and nerve cells.

14. A method for measuring cell **characterized by** using the device according to any one of claims 1 to 13.

15. A kit for measuring cell **characterized by** using the device according to any one of claims 1 to 13.
